(19) **Europäisches Patentamt**

**European Patent Office**

**Office européen des brevets**

(11) Publication number: **0 506 166 A2**

## (12) EUROPEAN PATENT APPLICATION

(21) Application number: **92200659.8**

(22) Date of filing: **09.03.92**

(51) Int. Cl.⁵: **A61K 31/70**, A23L 1/0522, A23L 1/308

(30) Priority: **25.03.91 EP 91200663**
**24.05.91 EP 91201232**

(43) Date of publication of application:
**30.09.92 Bulletin 92/40**

(84) Designated Contracting States:
**AT BE CH DE DK ES FR GB GR IT LI NL PT SE**

(71) Applicant: **UNILEVER N.V.**
**Burgemeester s'Jacobplein 1 P.O. Box 760**
**NL-3000 DK Rotterdam(NL)**

㊸ **BE CH DE DK ES FR GR IT LI NL PT SE AT**

(71) Applicant: **UNILEVER PLC**
**Unilever House Blackfriars P.O. Box 68**
**London EC4P 4BO(GB)**

㊸ **GB**

(72) Inventor: **van Amelsvoort , Johannes Mateus**
**Maria**
**Unilever Research Lab., Olivier van**
**Noortlaan 120**
**NL-3130 AC Vlaardingen(NL)**
Inventor: **Deckere, Emile Alphonsus**
**Unilever Research Lab., Olivier van**
**Noortlaan 120**
**NL-3130 AC Vlaardingen(NL)**
Inventor: **Kloots, Willem Jan**
**Unilever Research Lab., Olivier van**
**Noortlaan 120**
**NL-3130 AC Vlaardingen(NL)**

(74) Representative: **Roscoe, Brian Corrie et al**
**UNILEVER PLC Patents Division Colworth**
**House Sharnbrook**
**Bedford MK44 1LO(GB)**

(54) **Ingestible material.**

(57) Use of an effective amount of resistant starch as a blood cholesterol-reducing agent and/or blood triacylglycerol (or lipid) level-reducing agent in an ingestible material.

The present invention relates to ingestible materials having a specific effect on food digestion and absorption.

It is generally accepted that dietary fibres in human nutrition have favourable effects on gastro-intestinal functions and lipid and glucose metabolism. Usually, dietary fibre is measured as non-alpha-glucan polysaccharides (NSP) in plant foods. Detailed carbohydrate analysis showed that the apparent increase in NSP by food processing was due to a crystallized, short-chain alpha-glucan, which is essentially linear, and could be dispersed with potassium hydroxide, then hydrolyzed enzymatically and measured as glucose. This fraction was called resistant starch (RS).

It has been reported by K.M.Behall et al. in Am. J. Clin. Nutr., Vol. 49 (1989), pp. 337-344 and Vol. 50 (1989), pp. 1472-1474, that, when a basal diet comprising corn starch with either 70% of amylose (a straight-chain starch consisting solely of alpha-1,4 glucose linkages) or 70% of amylopectin (a branched-chain starch with both alpha-1,4 and alpha-1,6 glucose linkages) was fed to twelve volunteers for 5 weeks, the mean serum total cholesterol level and the mean fasting triacylglycerol (TAG) level were found to be significantly lower in the group on the amylose diet than in the group on the amylopectin diet. In the analysis and discussion of these findings, Behall et al. state that they were aware of the fact that amylose could yield considerable amounts of RS when cooked and cooled. They consider the amount of RS present in their diets not to be excessive, viz. 8.8% of RS after baking in the high-amylose muffins, and as a matter of fact they do not suggest that the reduced cholesterol level or the reduced blood TAG level in the group on the amylose diet either is caused by the presence of non-gelatinized starch or by the presence of RS.

This is confirmed in the more recent publication of J.M.Gee, R.M.Faulks and I.T.Johnson in J. Nutr. 121, 44-49 (1991) where it was concluded that there were no significant effects of dietary-resistant starch on serum cholesterol or blood TAG levels.

During investigations into the influence of RS in diets fed to Wistar rats, it has now been found that the presence of RS has a pronounced lowering effect on serum total cholesterol level and/or post-absorptive (sober) triacylglycerol (TAG) or lipid level.

Therefore, the present invention relates to the use of an effective amount of resistant starch as a serum cholesterol- and/or TAG-reducing agent in an ingestible material.

In general, the effective amount of resistant starch, based on the carbohydrate content of the ingestible material is such that the serum cholesterol and/or TAG reduction in the blood is clearly noticeable. Amounts of resistant starch, such as 20 to 25% by weight, based on the carbohydrate content of the ingestible material, have been found to be very effective, but also higher and lower amounts may effectively be used, such as from 5 to 90% by weight.

The ingestible material may also be formulated in such a way that, if only the blood TAG level is reduced, at the same time the blood cholesterol level will also be reduced or **vice versa**. This can be effected by, for example, adding soluble fibre material, such as pectins or guar gum. Furthermore, other additives, such as vitamins or minerals, may be present, provided that the positive blood TAG- or serum cholesterol-lowering effect is not detrimentally influenced by the presence of these other components.

As a source of resistant starch may be used : gelatinized starches having a high amylose content, such as maize or corn starch, sago, tapioca, potato starch, rice starch, and mixtures of these starches having a high percentage of resistant starch. The use of starches having about 20-25% of resistant starch is preferred.

The invention will be illustrated by the following Example.

EXAMPLE 1

A suspension of modified corn starch having an amylose content of 40% by weight and an amylopectin content of 60% by weight was made by suspending 41.6 g of this starch in 125 g of water, with stirring, at 95°C for 30 minutes. Thereafter the starch was fully digestible by alpha-amylase. The suspension was then cooled to 18°C, taking care that it remained homogeneous. The gelatinized mass was subsequently frozen to -18°C and kept at that temperature for at least 1 day. It was found to contain 23% by weight resistant starch.

In the same manner, non-modified corn starch (Maizena) was treated, except that this was heated at 70°C, with stirring, for 30 minutes. This non-modified corn starch was used to prepare the control diet (containing less than 2% by weight of resistant starch).

The gel obtained in both cases was used in the preparation of a diet consisting of :

|  | Energy % | g/MJ |
|---|---|---|
| Lard (37.7 kJ/g) | 20 | 5.3 |
| Calcium caseinate (15.72 kJ/g | 23 | 14.64 |
| Carbohydrate* (13.7 kJ/g) | 57 | 41.6 |
| Minerals | - | 0.86 |
| Vitamins | - | 0.2 |
| Cellulose | - | 3.6 |
| Water | - | 125.0 |

*) As a gel as prepared above with the indicated amount of water.

Two groups of male Wistar rats (n = 8), weighing about 300 g, were housed singly in cages with wire mesh bases and tops and placed in a room at 21°C with a 12 h light - 12 h dark cycle. All animals were trained for one week to receive two meals a day **ad libitum** between 07.00 and 07.30 hours and between 19.00 and 19.30 hours.

After the training, the animals received the diets as indicated above for a period of 3 weeks, i.e. group 1 received the diet with the modified high amylose corn starch and group 2 received the diet with the non-modified corn starch.

After these 3 weeks, 1 ml of tail blood was taken from the animals, just before the morning meal (t = 0 h) on Tuesday morning, and on Thursday one hour (t = 1 h) and two hours (t = 2 h) after the morning meal.

The level of total cholesterol (TC) and triacylglycerol (TAG)in the serum was determined enzymatically, the latter as total glycerol minus free glycerol.

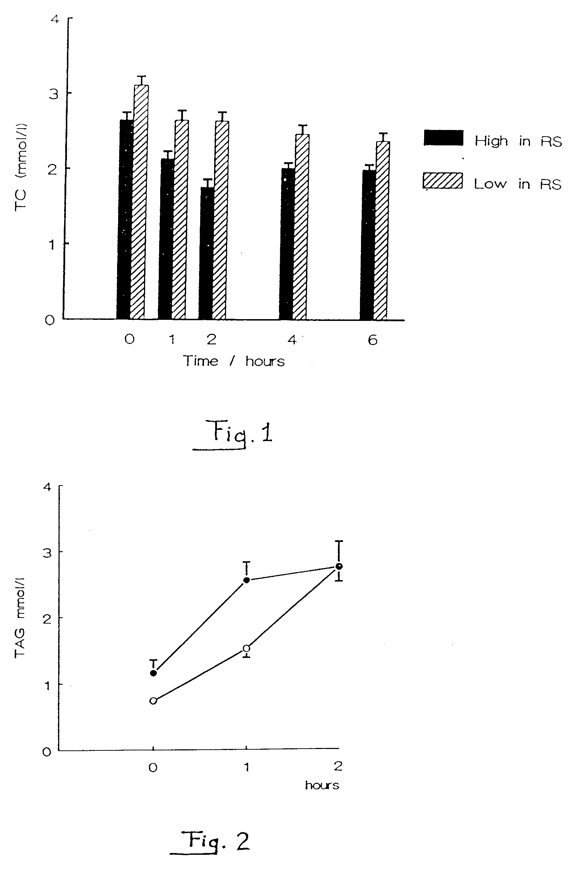

The total amounts of TC and TAG in mmol/l, just before (t = 0) and after the meal (t = 1 h and t = 2 h), found at the end of the 3-week diet period have been depicted in Fig. 1 and 2, respectively. In Fig. 1, the black bars indicate the amount of TC obtained with the diet high in resistant starch (RS) and the hatched bars indicate the amount of TC obtained with the diet low in resistant starch and in Fig. 2 the open circles indicate the amount of TAG obtained with the diet comprising the resistant starch and the closed circles indicate the amount of TAG obtained with the diet without the resistant starch.

From Fig. 1 it is clear that the ingestible material comprising the resistant starch leads to a reduction in the total cholesterol (TC) content in the blood upon consumption.

From Fig. 2 it is clear that in the sober phase there is a significant reduction in blood TAG level, which in the post-prandial period increases, but after a few hours decreases again to the low level observed in the sober phase.

## Claims

1. Use of an effective amount of resistant starch as a blood cholesterol and/or blood triacylglycerol (or lipid) level-reducing agent in an ingestible material.

2. Use of from 5% to 90% by weight, based on the carbohydrate content of the ingestible material, of resistant starch as a blood cholesterol level-reducing agent and/or blood triacylglycerol (or lipid) level-reducing agent in an ingestible material.

3. Use of about 20 to 25% by weight, based on the carbohydrate content of the ingestible material, of resistant starch as a blood cholesterol level-reducing agent and/or blood triacylglycerol (or lipid) level-reducing agent in an ingestible material.

Fig. 1

Fig. 2